Europäisches Patentamt

European Patent Office

Office européen des brevets

(11) Publication number: **0 335 263**
**A2**

(12) EUROPEAN PATENT APPLICATION

(21) Application number: 89105227.6

(22) Date of filing: 23.03.89

(51) Int. Cl.4: **C12N 5/00 , C12P 21/00 , C12N 15/00 , C07K 15/06 , G01N 33/68 , //(C12P21/00, C12R1:91)**

A request for correction of the description has been filed pursuant to Rule 88 EPC. A decision on the request will be taken during the proceedings before the Examining Division (Guidelines for Examination in the EPO, A-V, 2.2).

The microorganism(s) has (have) been deposited with Fermentation Research Institute under number(s) BP-1813, BP-1814, BP-1587, BP-1589, BP-1591.

(30) Priority: 29.03.88 JP 73155/88

(43) Date of publication of application:
04.10.89 Bulletin 89/40

(84) Designated Contracting States:
AT BE CH DE ES FR GB GR IT LI LU NL SE

(71) Applicant: TAKEDA CHEMICAL INDUSTRIES, LTD.
3-6, Doshomachi 2-chome Chuo-ku
Osaka 541(JP)

(72) Inventor: Iwasa, Susumu
21-2, Ohsumigaoka 1-chome Tanabe-cho
Tsuzuki-gun Kyoto 610-03(JP)
Inventor: Nakata, Mitsugu
22-5, Kiyomidai 1-chome Kawachinagano
Osaka 586(JP)
Inventor: Toyoda, Yukio
8-8, Mizudocho 1-chome Amagasaki
Hyogo 661(JP)

(74) Representative: von Kreisler, Alek et al
Patentanwälte Von
Kreisler-Selting-Werner-Schönwald-Fues-Da-
llmeyer Deichmannhaus
D-5000 Köln 1(DE)

(54) Anti-human lymphotoxin monoclonal antibody, hybridoma for production thereof, anti-human lymphotoxin-C-terminal peptide antibody and method for detecting human lymphotoxin thereby.

(57) Disclosed are a human lymphotoxin-neutralizing monoclonal antibody-producing hybridoma, an anti-human lymphonotoxin monoclonal antibody produced by that hybridoma and an anti-human lymphotoxin neutralizing antibody. The hybridoma is obtained by fusing a myeloma cell with a spleen cell or a lymphonodus cell of an animal immunized with an N-terminal-deleted human lymphotoxin. The anti-human lymphotoxin neutralizing antibody is harvested from an animal immunized with a human LT-C-terminal peptide.

The two kinds of antibodies described above can be used for an immunochemical method for assaying a human lymphotoxin in a test solution, one as an antibody to be held on a carrier and the other as an antibody to be linked with a labeling agent.

# ANTI-HUMAN LYMPHOTOXIN MONOCLONAL ANTIBODY, HYBRIDOMA FOR PRODUCTION THEREOF, ANTI-HUMAN LYMPHOTOXIN-C-TERMINAL PEPTIDE ANTIBODY AND METHOD FOR DETECTING HUMAN LYMPHOTOXIN THEREBY

Background of the Invention

The present invention relates to a monoclonal antibody (hereinafter occasionally referred to as MoAb) against human lymphotoxin (hereinafter occasionally referred to as LT), a hybridoma for producing a MoAb, and a polyclonal antibody (hereinafter occasionally referred to as PoAb) against human LT-C-terminal peptide. The invention is further directed to an immunochemical detecting method for assaying human LT with high sensitivity by use of the two kinds of antibodies described above.

LT is a protein derived from lymphocytes, which consists of 171 amino acid residues, and is believed to be an antitumor agent in a manner similar to tumor necrosis factor (hereinafter referred to as TNF) derived from macrophages, due to its selective antitumor activity. The mass production of recombinant proteins has recently become possible [Nature, 312, 721 and 724 (1984)]. From the clinical applications of recombinant TNF and LT, some therapeutic effects have been observed [Oncologia, 20, 105 (1987)].

However, even with such technical progress and in order to increase the number of clinical trials, it has become an important issue to establish a method for extraction and purification of the recombinant human LT from microorganisms or cell cultures and a method for assaying for small quantities of human LT in clinical specimens such as sera and urine with high sensitivity. An antibody reactive to human LT and an enzyme-linked immunosorbent assay (hereinafter occasionally referred to as ELISA) using the antibody have been reported by Bringman et al. [Hybridoma, 6 489 (1987)]. They used natural human LT and recombinant human LT each consisting of 171 amino acid residues as a main component of an immunogen for preparation of the antibody. However, with respect to human LT, it is believed that the N-terminal region, which is independent of biological activity, is the site having the highest immunogenicity. When an animal is immunized with an antigen having human LT (1-171) as a main component, antibodies to the N-terminal having no neutralization activity are produced in large quantity. According to Bringman et al., although 13 kinds of mouse monoclonal antibodies were obtained, 7 of them were N-terminal antibodies having no neutralization activity. Further, the detection range of human LT by a sandwich (hereinafter occasionally referred to as SW)-ELISA using rabbit PoAb obtained by the immunogen described above is 0.4 to 25 ng LT/ml, which is not necessarily satisfactory. A SW method in which human LT of an antigen to be determined is sandwiched by two kinds of antibodies is an excellent assay system less susceptible to disturbance by non-specific components in a test solution. However, improper combination of two kinds of antibodies to be used often results in an decrease of the sensitivity. In order to obtain a good correlation between determinations by a bioassay and those by an ELISA, it is desirable to use suitable neutralizing antibodies.

In this specification, amino acids and peptides are indicated by the abbreviations adopted by IUPAC-IUB Committee of Biochemistry Nomenclature (CBN). For example, the following abbreviations are used:

Gln: Glutamine residue
Asp: Aspartic acid residue
Pro: Proline residue
Tyr: Tyrosine residue
Val: Valine residue
Lys: Lysine residue
Glu: Glutamic acid residue
Ala: Alanine residue
Asn: Asparagine residue
Leu: Leucine residue
Phe: Phenylalanine residue
Gly: Glycine residue
His: Histidine residue
Ser: Serine residue
Thr: Threonine residue
Ile: Isoleucine residue
Trp: Tryptophan residue
Arg: Arginine residue
Met: Methionine residue

When the optical isomer is capable of existing with respect to the amino acids and the like, the L-form is represented unless otherwise specified.

Also in this specification, polymers or oligomers of deoxyribonucleic acid (hereinafter referred to as DNA) are indicated by the sequence of the following abbreviations:

A: 2'-Deoxyadenylic acid residue
C: 2'-Deoxycytidylic acid residue
G: 2'-Deoxyguanylic acid residue
T: Thymidylic acid residue

Unless otherwise stated, the direction from the left to the right in sequence indicates the direction from 5'-position to the 3'-position.

Brief Description of the Drawings

Fig. 1 is a graph showing human LT standard curves; and

Fig. 2 is a graph showing the comparison of determinations of human LT contained in a test solution by an ELISA with those by a bioassay.

Disclosure of the Invention

The present inventors have discovered that anti-human LT antibody-producing hybridoma cells having high neutralization activity can be prepared by producing an N-terminal deleted human LT mutein having high antitumor activity by genetic engineering technique, immunizing mice with the resultant mutein as an immunogen, and harvesting the hybridoma cells from their spleens. They further discovered that an anti-human LT antibody having very high neutralization activity can be unexpectedly obtained by conjugating a synthetic peptide of a human LT-C-terminal portion to a carrier protein and immunizing rabbits therewith. Furthermore, the present inventors have found that the SW-ELISA using the two kinds of anti-human LT antibodies described above in combination with each other can specifically determine with high sensitivity only human LT having biological activity. Further studies based upon these findings have resulted in completion of this invention.

That is to say, the present invention relates to a hybridoma producing a human lymphotoxin neutralizing monoclonal antibody, obtainable by fusing a myeloma cell with a spleen cell or a lymphonodus cell of an animal immunized with an N-terminal-deleted human LT having the following amino acid sequence, and to a monoclonal antibody produced by that hybridoma:

H-(Met)n-R₁-R₂-Ala-His-Ser-Thr-Leu-Lys-Pro-Ala-Ala-His-Leu-Ile-Gly-Asp-Pro-Ser-Lys-Gln-Asn-Ser-Leu-Leu-Trp-Arg-Ala-Asn-Thr-Asp-Arg-Ala-Phe-Leu-Gln-Asp-Gly-Phe-Ser-Leu-Ser-Asn-Asn-Ser-Leu-Leu-Val-Pro-Thr-Ser-Gly-Ile-Tyr-Phe-Val-Tyr-Ser-Gln-Val-Val-Phe-Ser-Gly-Lys- Ala-Tyr-Ser-Pro-Lys-Ala-Thr-Ser-Ser-Pro-Leu-Tyr-Leu-Ala-His-Glu-Val-Gln-Leu-Phe-Ser-Ser-Gln-Tyr-Pro-Phe-His-Val-Pro-Leu-Leu-Ser-Ser-Gln-Lys-Met-Val-Tyr-Pro-Gly-Leu-Gln-Glu-Pro-Trp-Leu-His-Ser-Met-Tyr-His-Gly-Ala-Ala-Phe-Gln-Leu-Thr-Gln-Gly-Asp-Gln-Leu-Ser-Thr-His-Thr-Asp-Gly-Ile-Pro-His-Leu-Val-Leu-Ser-Pro-Ser-Thr-Val-Phe-Phe-Gly-Ala-Phe-Ala-Leu-OH

wherein R₁ is Pro or Phe, R₂ is a peptide chain represented by Ala-Gln-Thr-Ala-Arg-Gln-His-Pro-Lys-Met-His-Leu or a part thereof, and n is 0 or 1 is preferred. However, substitutions, deletions and/or additions to the above-described amino acid sequence can be used as long as the resultant N-terminal deleted human LT retains its biological activity or produces an antibody to human LT.

Further, the present invention relates to an anti-human LT neutralizing antibody which can be harvested from an animal immunized with a human LT-C-terminal peptide having the following amino acid sequence:

H-R-Ser-Thr-Val-Phe-Phe-Gly-Ala-Phe-Ala-Leu-OH

wherein R is a peptide chain represented by Leu-Thr-Gln-Gly-Asp-Gln-Leu-Ser-Thr-His-Thr-Asp-Gly-Ile-Pro-His-Leu-Val-Leu-Ser-Pro or a part thereof.

Furthermore, the present invention is directed to an immunochemical method, preferably ELISA, for assaying human LT using the two kinds of antibodies described above as an antibody to be held on a carrier and an antibody to be linked with a labeling agent, respectively. One of the two kinds of antibodies described above includes, for example, a mouse monoclonal neutralizing antibody such as LT3-11 or LT3-135, and the other includes, for example, rabbit neutralizing antibody such as LT-R1, LT-R2 or LT-R3.

In preparing the monoclonal anti-human LT antibody-producing hybridoma of the present invention, N-terminal deleted LT is used as an immunogen. Therefore, the DNA coding for N-terminal-deleted LT is prepared, for example, by the following process:

1. m-RNAs can be collected by known methods from human peripheral blood lymphocytes in which the synthesis of LT is induced by 12-o-tetradecanoylphorbol-13-acetate (TPA) and concanavalin A (ConA). Further, about 5 X 10⁵ cDNA library can be prepared therefrom.

2. An oligonucleotide from 10-mer to 50-mer coding for a partial peptide chain of LT is synthesized. By using this oligonucleotide as a probe, the screening of LT cDNA can be conducted. For example, when a synthetic nucleotide of 18-mer (TCCAAAGAAGACAGTACT) at the C-terminal side is used, about 50 clones can be obtained.

3. Plasmids are isolated from the LT cDNA clones thus obtained and the nucleotide sequence is determined. A plasmid coding for the amino acid sequence of LT described above is selected, cleaved with an appropriate restriction enzyme, and then suitably inserted into an expression vector by known techniques. Thus, a recombinant DNA containing the DNA fragment can be prepared.

4. Some kinds of hosts, for example, Escherichia coli, are transformed by using the vector prepared in item 3. Consequently, strains containing the DNA coding for LT can be obtained.

5. After the transformants prepared in 4 are cultivated and the plasmids are isolated, the DNA coding for N-terminal-deleted LT is prepared as follows:

(1) In the case of the human LT gene, a restriction enzyme NsiI recognition site is located in the region coding for methionine-histidine situated in the 20th-21st positions from the N-terminus. A DNA fragment coding for the N-terminal-deleted LT can be obtained by digesting the LT gene with NsiI at that site. A DNA fragment coding for LT (20-171) is produced by linking an appropriate adapter containing the following sequence with the above fragment and the produced DNA fragment is inserted into an appropriate vector:

```
5'                              3'
    G A A T T C A T G C C
    C T T A A G T
3'                      5'
```

(2) A chemically synthesized DNA fragment is linked with the DNA fragment coding for N-terminal-deleted LT which has been cleaved with NsiI. On this occasion, a DNA fragment coding for a peptide chain corresponding to the chain from alanine situated in the 10th position to methionine situated in the 20th position from the N-terminus, or a DNA fragment coding for a peptide chain in which an amino acid or peptide of a part of the above peptide chain is eliminated or substituted with another amino acid or peptide can be used. These may be synthesized so as to maintain the reading frame correctly.

(3) Similarly, in the case of the human LT gene, a restriction enzyme PvuII recognition site is located in the region coding for serine-alanine situated in the 9th-10th positions from the N-terminus. A DNA fragment coding for N-terminal deleted LT can be obtained by digesting the LT gene with PvuII. A DNA fragment coding for LT (10-171) is produced by linking an appropriate adapter containing the following sequence with the above fragment and the produced DNA fragment is inserted into an appropriate vector:

```
5'                          3'
    G A A T T C A T G C
    C T T A A G T A C G
3'                      5'
```

(4) The DNA fragment coding for N-terminal deleted LT which has been cleaved with PvuII is further treated with an exonuclease such as nuclease BAL31 to remove the region coding for 1-10 amino acids therefrom. Then, a DNA fragment coding for LT (X-171), X = 10 to 20, can be prepared by selecting the DNA fragment in which the reading frame of the gene is correctly maintained.

(5) The technique of site-directed mutagenesis [M. Smith and S. Gillam, Genetic Engineering, 3, 1 (1981)] may also be applied. Namely, after cleavage with PvuII, the DNA fragment coding for N-terminal-deleted LT is inserted into vector M13, with which Escherichia coli JM103 (Pharmacia P-L Biochemicals) is infected. After cultivation, M13 phages released in the broth are precipitated with polyethylene glycol and treated with phenol, whereby a single stranded M13 phage DNA can be obtained.

Then, the DNA coding for a peptide chain in which an amino acid or peptide of a part of the peptide chain from alanine situated in the 10th position to methionine situated in the 20th position from the N-terminus of LT is eliminated or substituted with another amino acid or peptide is prepared by a chemical synthesis method and used as a primer. This primer is mixed with the M13 phage DNA fragment previously prepared to form a double-stranded DNA by DNA polymerase I large fragment. Thereafter, the cyclization thereof can be achieved by T4 DNA ligase. This circular DNA is introduced into Escherichia coli JM103, and the released M13 phage DNAS are transferred onto a filter. Then, plaque hybridization [T. Maniatis et al., Molecular Cloning, Cold Spring Harbor Laboratory, p.312 (1982)] is carried out by using the synthesized primer labeled with $^{32}$P. A DNA is prepared from the phage from which a strong signal is detected, and cut out with an appropriate restriction enzyme. The DNA fragment thus obtained is inserted into a plasmid to provide a DNA coding for modified N-terminal-deleted LT.

Plasmid DNA, cosmid DNA, phage DNA and the like containing the whole or a part of the human LT gene can be used as a template DNA for the site-directed mutagenesis. M13 phage or φX174 phage is desirable as a template DNA, because a single-stranded DNA can be easily prepared therefrom. For example, when M13 phage or φX174 phage in which the whole or a part of the human LT gene is inserted is used, phage particles present in the broth are precipitated with polyethylene glycol, deproteinized by phenol treatment, and then precipitated with ethanol to obtain a single-stranded DNA in the phage particle. When a double-stranded plasmid DNA or cosmid DNA in which the whole or a part of the human LT gene is inserted is used as a template DNA, the double-stranded DNA is subjected to heat treatment at 100°C for 1 to 10 minutes, preferably 3 to 5 minutes, and then cooled rapidly with ice water to be denatured to a single-stranded DNA, prior to the use.

The primer for the site-directed mutagenesis may have any DNA sequence so long as it has a DNA sequence to be changed and functions as the primer on the DNA synthesis through hybridization with a template DNA. The primer may be produced by any method, but it is desirable to prepare a single-stranded DNA having an appropriate sequence by a chemical synthesis method. Such a primer is mixed with the single-stranded DNA previously prepared, and repaired to a double-stranded DNA by DNA polymerase I large fragment. Thereafter, the cyclization can be achieved by T4 DNA ligase. After the circular DNA thus obtained is introduced into Escherichia coli, plaque hybridization [T. Maniatis et al., Molecular Cloning, Cold Spring Harbor Laboratory, p.312 (1982)] or colony hybridization (ibid. p. 326) is carried out, using the primer labeled with a radioisotope as a probe, whereby the desired mutant can be selected. By using phage DNA or plasmid DNA prepared from the plaque or the colony thus obtained, an N-terminal-deleted LT gene can be prepared.

Then, a recombinant DNA capable of expressing the DNA coding for N-terminal deleted LT can be constructed, by inserting the obtained DNA fragment coding for N-terminal-deleted LT at the 3′-terminus of the promoter region which functions in the host to be used. Hosts include Escherichia coli, Bacillus subtilis, yeast or animal cells.

Any promotor region is available, if the region contains a site necessary for initiating the mRNA synthesis by linkage of RNA polymerase.

For example, when Escherichia coli is used as a host, the recombinant DNA capable of expressing the DNA sequence coding for N-terminal-deleted LT can be constructed by inserting the DNA fragment coding for N-terminal-deleted LT at the 3′-terminus of a promoter region capable of functioning in Escherichia coli. When Escherichia coli is employed as the host in this way, pBR322, pBR325, ptrp781, pUC8, pUC9, pJB8 or the like can be used as the vector, and the DNA fragment coding for N-terminal deleted LT is inserted therein by T4 DNA ligase. By using this reaction mixture, Escherichia coli such as C600 strain, MM294 strain, DH1 strain, W3110 strain, PR1 strain or PR13 strain is transformed according to the method of S. N. Cohen et al., Proc. Natl. Acad. Sci. U.S.A., 69, 2110 (1972) or a similar method thereto.

The promoter used is not limited to the trp promoter (trp-p). For example, there may be used the recA promoter (Japanese Patent Unexamined Publication No. 59- 65099/1984), the lac promoter, the λP$_L$ promoter or the like.

The transformant carrying the recombinant plasmid DNA containing the DNA fragment coding for N-terminal-deleted LT obtained as described above can be selected by a phenotype, for example, an ampicillin resistance type, a tetracycline resistance type or a resistance type to both of these drugs.

The transformant described above is cultivated in a medium which is known per se. The media include, for example, L-broth, Penassay broth and M-9 medium containing glucose and Casamino Acids [J. Miller, Experiments in Molecular Genetics, p. 431-433 (Cold Spring Harbor Laboratory, New York, 1972)]. In order to allow the promoter to act efficiently, a drug such as 3β-indolylacrylic acid may be added thereto if necessary.

Cultivation of the above transformant is generally carried out at 15 to 43°C, preferably 28 to 40°C, for 2 to 24 hours, preferably 4 to 16 hours, with aeration or agitation if necessary.

After cultivating, the cells are collected by a known method. In case of the transformant of Escherichia coli, the cells are suspended in an appropriate buffer, for example, Tris-hydrochloric acid buffer (pH 7.5), and disrupted by ultrasonic treatment, lysozyme and/or freeze-thawing. Thereafter, the supernatant containing N-terminal deleted LT is obtained by centrifugation. There can be preferably employed a method in which the collected cells are suspended in a buffer solution, to which lysozyme is added, and incubated at 0 to 10°C for 10 minutes to 3 hours, and the resulting culture is treated with ultrasonication at 0 to 10°C for 30 seconds to 5 minutes and then centrifuged to obtain the supernatant.

Separation and purification of N-terminal deleted LT from the extract can be carried out, for example, by gel filtration, hydroxyapatite column chromatography, ion-exchange column chromatography, ultracentrifugation or affinity chromatography using human LT antibody.

There are hereinbefore described the methods for obtaining the gene of N-terminal deleted LT and for producing N-terminal deleted LT by using the gene. However, the methods are not limited to those methods, which are only exemplary.

The N-terminal-deleted LT described above is inoculated in animals to induce an anti-human LT antibody. The animals for inoculation include, for example, rabbit, rat, mouse and guinea pig. When MoAb is prepared, mouse is preferably used.

The inoculation is performed according to a standard method. There is adopted, for example, a method in which 1 to 100 μg each time, preferably 10 to 25μg each time of the N- terminal deleted LT emulsified with the same volume (0.1 ml) of a physiological saline and Freund's complete adjuvant is inoculated on a mouse subcutaneously at the back or the abdomen or intraperitoneally, 3 to 6 times every 2 to 3 weeks.

Individuals having a high antibody titer are selected from these immunized animals such as mice, and their spleens or lymphatic corpuscles are collected therefrom 3 to 5 days after the final immunization. Then, antibody-producing cells contained therein are fused with myeloma cells. The fusing operation may be conducted according to a known method. Fusion accelerators include polyethylene glycol (hereinafter referred to as PEG) and Sendai virus. In particular, PEG is preferably used. The myeloma cells include NS-1, P3UI and Sp2/0. Particularly, P3UI is preferably used. The preferable ratio of the number of the spleen cells to that of the myeloma cells is 1:1 to 10:1. It is preferred that PEG having a polymerization degree of 1,000 to 6,000 is added thereto in a concentration of 10 to 80% and the resulting mixture is incubated at 20 to 37°C, preferably 30 to 37°C, for 3 to 10 minutes.

The human LT antibody-producing hybridoma cells can be screened by various methods. For example, there is applied an ELISA which comprises adding the hybridoma culture supernatant to a microtiter plate on which human LT is adsorbed, then adding the anti-mouse immunoglobulin antibody labeled with horseradish peroxidase (hereinafter referred to as HRP) thereto, and detecting the anti-human LT monoclonal antibody bound to the plate as the solid phase. Hybridoma cells having positive antibody activity are immediately subjected to cloning, which is usually conducted with ease by a limiting dilution method. The antibody titer of the cloned hybridoma culture supernatant is determined by the above-mentioned method and hybridoma cells which stably produce antibody having a high titer are selected. Thus, the desired monoclonal hybridoma cells can be obtained.

Examples of the anti-human LT antibody-producing hybridoma cells prepared according to the above methods include mouse hybridoma LT3-11 and LT3-135 shown in Example 1 which will hereinafter be described.

Then, on preparation of the antibody against the human LT-C-terminal peptide of the present invention, a human LT-C-terminal peptide is chemically synthesized as an immunogen. The above C-terminal peptide is prepared by a known conventional peptide synthesis method such as a solid synthesis method or a liquid synthesis method. Such peptide synthesis methods include, for example, the methods described in Schroder and Lubke, The Peptides, vol. 1, Academic Press, New York, U.S.A. (1986) or Izumiya et al., Peptide Synthesis, Maruzen (1975), such as the azide method, the chloride method, the acid anhydride method, the mixed anhydride method, the DCC method, the active ester method, the method using Woodward reagent K, the carbodiimidazole method, the redox method and DCC/additive (for example, HONB, HOBt or HOSu) method.

The peptide thus obtained is conjugated with a carrier protein such as bovine serum albumin (hereinafter referred to as BSA), bovine thyroglobulin (hereinafter referred to as BTG) or ovalbumin (hereinafter referred to as OVA), and then inoculated on animals. The animals for inoculation include, for example, rabbit, sheep, goat, rat, guinea pig and mouse. Inoculation is often performed, for example, according to a method in which 1 to 2mg each time of the C-terminal peptide emulsified with the same volume (1 ml) of a physiological saline and Freund's complete adjuvant is inoculated on an animal subcutaneously at the back or the hind-limb palm, 4 to 6 times every 3 to 4 weeks to produce the antibody. After 7 to 12 days from the final immunization, the blood is collected from the aural veins to obtain the serum containing the anti-human LT antibody.

The anti-human LT-C-terminal peptide antibodies prepared according to the above methods include rabbit antibody LT-R1, LT-R2 and LT-R3 shown in Example 2 which will hereinafter be described.

In the immunochemical method for assaying human LT according to the present invention, there is employed a so-called sandwich (SW) method using two kinds of antibodies wherein their antigen-determining sites do not overlap. The principle of the sandwich method is as follows:

An excess of antibody held on a carrier is added to a test solution containing an unknown amount of an antigen, followed by reaction (the first reaction). Then, a definite and excess amount of an antibody labeled with a labeling agent is added thereto, followed by reaction (the second reaction). The activity of the labeling agent held or not held on the carrier is assayed. The first and second reactions may be conducted at the same time or at different times.

This SW method is generally less susceptible to disturbance by non-specific components other than LT in the test solution than a competitive method, and enables the assay to be performed in a short time. In the present invention, the antibody on the solid phase used in the first reaction and the labeled antibody used in the second reaction are two kinds of hetero-derived antibodies wherein their antigen-determining sites do not overlap.

In order to prepare the antibody on the solid phase, there can be applied known conventional methods which include, for example, the cyanogen bromide method and glutaraldehyde (hereinafter referred to as GLA) method described in Metabolism, 8, 696 (1971). Simpler and more preferable methods include a method in which the antibody is adsorbed on a microplate or polystyrene particles.

The labeling agents applied to the labeled antibody used in the second reaction include radioisotopes (hereinafter referred to as RI), enzyme, enzyme substrates, fluorescent substances, luminescent substances and biotin. In order to link the antibody with the labeling agent, there are used known conventional methods which include the chloramine T method [Nature, 194, 495 (1962)], the periodate acid method [J. Histochem. Cytochem., 22, 1084 (1974)], the maleimide method [J. Biochem., 79, 233 (1976)] and the activated biotin method [J. Am. Chem. Soc., 100, 3585 (1978)].

When the specific immunochemical method for assaying human lymphotoxin is carried out according to the SW method, a solid phase to which an antibody is physically or chemically bound by a known conventional method is added to a test solution containing an unknown amount of human LT, followed by reaction (the first reaction). After the solid phase is washed, a definite amount of another antibody labeled with a labeling agent is added thereto, followed by reaction (the second reaction). Then, the solid phase is further thoroughly washed, and thereafter the activity of the labeling agent bound to the solid phase is assayed. When the labeling agent is a RI, a well counter or a liquid scintillation counter is used for determination. When the labeling agent is an enzyme, a substrate is added thereto and the mixture is allowed to stand. Then, the enzyme activity is determined by a colorimetry or a fluorescence method. Even if the labeling agent is a fluorescent substance or a luminescent substance, a known method is employed for each determination. In the assay described above, the washing between the first reaction and the second reaction may be omitted. For further simplifying the procedure, the test solution, the antibody-bound solid phase and the antibody labeled with the labeling agent may be added together to react them with one another. Namely, the antibodies used in the present invention have a very excellent feature that the reaction is less influenced by the order of addition of the reagents, the time of addition thereof, the presence or absence of the washing and the like, due to their different antigen-determining sites.

The present invention will hereinafter be described in detail with the following Reference Examples and Examples. It is understood of course that these Reference Examples and Examples are not intended to limit the scope of the invention.

In carrying out the present invention, preparation of recombinant DNA and insertion of the recombinant into the microorganism were conducted according to the following experimental textbooks unless otherwise stated:

(1) T. Maniatis, E. F. Fritsch and J. Sambrook, Molecular Cloning, published by Cold Spring Harbor Laboratory (U.S.A.)

(2) Edited by Y. Takagi, Gene Manipulation Experimental Method, published by Kodansha (Japan)

Mouse-mouse hybridomas LT3-11 and LT3-135 disclosed in the Examples are deposited in the Institute for Fermentation (IFO), Osaka, Japan with accession numbers IFO 50167 and IFO 50164, respectively, and also deposited in the Fermentation Research Institute, Agency of Industrial Science and Technology, Ministry of International Trade and Industry (FRI), Japan with accession number FERM BP-1813 and FERM BP-1814, respectively.

Escherichia coli DH1 and C600 strains carrying plasmids pTB618, pTB622 and ptrp781 (Escherichia coli DHI/pTB618, Escherichia coli C600/pTB622 and Escherichia coli DH1/ptrp781) are depsoited in IFO with accession numbers IFO 14542, IFO 14544 and IFO 14546, respectively, and also deposited in FRI with accession numbers FERM BP-1587, FERM BP-1589 and FERM-1591, respectively.

Reference Example 1

Evaluation of Cytotoxic Activity against L929 Cells

The cytotoxicity of LT was measured by a method similar to that described in J. Immunol., 126, 235 (1981) or J. Immunol. Methods, 70, 257 (1984), using L929 cells. Namely, 50 $\mu$l of L929 cells suspended in a concentration of 4 X $10^5$ cells/ml in RPMI 1640 medium supplemented with 10% fetal calf serum (FCS) containing 4 $\mu$g/ml of mitomycin C were added to 50 $\mu$l of a sample serially two-fold diluted with RPMI medium supplemented with 10% FCS by using a microplate for tissue culture (Flow Laboratory) having 96 wells, and then cultivated in 5% carbonic acid gas at 37° C for 48 hours. After completion of the cultivation, the live cells were dyed with dimethylthiazolyl diphenyltetrazolium bromide (MTT), and dissolved with 10% SDS-0.01N HCl. Thereafter, the absorbance at 590 nm was measured with Titertek Multiscan (Flow Laboratory). The absorbance obtained is proportional to the number of live cells. The amount of biological activity required for killing 50% of L929 cells was defined as 1 unit/ml, and the biological activity of the sample was represented by unit/ml.

Reference Example 2

Collection of Natural LT

In accordance with the method described in Hinuma et al., Microbiol Immunol., 28, 935 (1984), normal human peripheral blood lymphocytes activated by TPA and ConA were cultivated in RPMI 1640 medium containing 10% FCS in 5% carbon dioxide gas at 37°C for 2 to 3 days, and a solution containing LT was obtained from the culture supernatant.

Reference Example 3

Preparation of Escherichia coli Strains for Transformation

Each colony of Escherichia coli strains DHI, C600 and MM294 was cultivated in 10 ml of SOB medium [experimental textbook (1), p. 69], until an absorbance at 550 nm reached to 0.3. After being cooled with ice, the culture was centrifuged. Then, the resulting cells were washed with 5 ml of 10 mM NaCl. The cells were resuspended in 5 ml of 50 mM CaCl₂ and allowed to stand for 15 minutes under ice cooling. After centrifugation, the cells were suspended in 0.5 ml of 50 mM CaCl₂ and used immediately thereafter.

Reference Example 4

Preparation of N-Terminal Deleted Human Lymphotoxin

(1) Preparation of cDNA Library by Using mRNA Derived from Human Lymphocytes.

The lymphocytes prepared from human peripheral blood were cultivated in RPMI 1640 medium (supplemented with 10% FCS) containing TPA (15 ng/ml) and ConA (40 μg/ml) at 37°C to induce LT. After 24 hours, 1 X 10¹⁰ cells of the induced human lymphocytes were denatured by disruption with a Teflon homogenizer in a solution containing 5 M guanidine thiocyanate, 5% mercaptoethanol, 50 mM Tris-HCl (pH 7.6) and 10 mM EDTA. Thereafter, sodium N-lauroylsarcosinate was added thereto to a concentration of 4%, and the homogenized mixture was layered on 6 ml of 5.7 M cesium chloride solution (5.7 M cesium chloride and 0.1 M EDTA), and then centrifuged by a Beckmann SW28 rotor at 24,000 rpm. at 15°C for 48 hours, whereby the precipitate of RNA was obtained. After this precipitate of RNA was dissolved in 0.25% sodium N-lauroylsarcosinate solution, precipitation with ethanol was carried out, whereby 10 mg of RNA was obtained. The RNA thus obtained was adsorbed on an oligo(dT) cellulose column in a high salt solution [0.5 M NaCl, 10 mM Tris-HCl (pH 7.6), 1 mM EDTA and 0.3% SDS], and eluted with a low salt solution [10 mM Tris-HCl (pH 7.6), 1 mM EDTA and 0.3% SDS], to obtain 300 μg of mRNA containing poly(A).

The mRNA thus obtained was further precipitated with ethanol, and dissolved in 0.2 ml of a solution [10 mM Tris-HCl (pH 7.6), 2 mM EDTA and 0.3% SDS]. After treatment at 65°C for 2 minutes, fractionation by sucrose density gradient centrifugation (by a Beckmann SW28 rotor, at 25,000 rpm at 20°C for 21 hours) was conducted. For each of the fractions, a part of RNA was injected into oocyte cells of Xenopus laevis, and the LT activity in protein to be synthesized was measured. As a result, the activity of LT was detected in the fraction corresponding to a sedimentation constant of about 16S. The amount of LT mRNA in this fraction was about 25 ug.

Using the poly(A) RNA as a template, a cDNA library was prepared by using pcDVL vector and pL1 linker according to the method of Okayama and Berg [Mol. Cell. Biol., 2, 161 (1982); ibid., 3, 280 (1983)]. Escherichia coli DH1 was infected with circular vector plasmids containing cDNA, and a cDNA library of about 5 X 10⁵ clones of which the host was Escherichia coli DH1 was obtained from 5 μg of poly(A) RNA.

(2) Isolation of Plasmid Containing Human LT cDNA and Determination of Nucleotide Sequence Thereof

The above human cDNA library using Escherichia coli DH1 was inoculated to 10 nitrocellulose filters (HATF filter manufactured by Millipore) so as to reach 3 X 10⁴ clones/filter. Twenty (20) replica filters (each pair consisting of 2 filters) were prepared from the above 10 master filters. Plasmid DNA exposed for denaturation by lysing Escherichia coli on the replica filter with 0.5 N NaOH solution was dried for fixation on the filter [M. Grunstein and D. S. Hogness, Proc. Natl. Acad. Sci. U.S.A., 72, 3961 (1975)].

An oligonucleotide having the following formula, which corresponded to a portion (the gene portion corresponding to amino acid No. 162-167) of the nucleotide sequence of the LT gene already reported [Gray et al., Nature, 312, 721 (1984)], was synthesized and used as a screening probe for human LT cDNA.

⁵TCCAAAGAAGACAGTACT³

The 5′-terminus of the oligonucleotide probe

was labeled with $^{32}P$ by using T4 polynucleotide kinase and $[\gamma-^{32}P]ATP$.

The labeled probe was hybridized with each of the replica filters on which DNA was fixed. The hybridization reaction was carried out at 40°C for 16 hours in 10 ml of a solution of 5 X SSC (0.15 M NaCl, 0.015 M sodium citrate), 5 X Denhardt's, 0.1% SDS and 100 μg/ml of denatured salmon sperm DNA containing 10 μCi of the labeled probe. After completion of the reaction, the filters were washed with a solution of 6 X SSC and 0.1% SDS at room temperature for 30 minutes 3 times and further at 43°C for 60 minutes twice [the experimental textbook (1), p. 309]. Radioautograms were taken from the washed filters and the radioautograms of the replica filters in sets of two filters were put together in layers for searching the cells reactive to the probe. By this method, 50 Escherichia coli DH1 strains reactive to the probe were obtained from about 3 X 10⁵ colonies.

The plasmid DNA was extracted and purified from these cells by the alkaline method [C. H. Birnboim and J. Doly, Nucleic Acids Res., 11, 1513 (1979)]. The DNA was cleaved by the restriction enzyme BamHI (Takara Syuzo) and fractionated by agarose gel electrophoresis. Thereafter, the DNA fragments were transferred on a nitrocellulose filter (BA85 manufactured by S & S) [Southern blotting method, the experimental textbook (1), p. 382]. When this filter was hybridized with the oligonucleotide probe described above, the plasmid DNA fragments reacted with the probe.

Then, one strain of Escherichia coli K12 DH1/pTB618 having the plasmid which had the largest BamHI DNA fragments (cDNA portions) among others was selected. The nucleotide sequence of the cDNA portion of this plasmid DNA was determined by the dideoxynucleotide synthetic chain termination method [J. Messing et al., Nucleic Acids Res., 9, 309 (1981)].

As a result, it was proved that the LT gene contained in plasmid pTB618 was not complete and included from the non-translated portion on the 3′-terminal side upstream to the third C of codon CCC of Pro which was the 18th amino acid.

(3) Construction of Human LT (21-171) Expression Vector pTB622

The plasmid pTB618 prepared above was cleaved by restriction enzymes NsiI (Takara Syuzo) and BamHI, and a DNA fragment of 1.1 kilo base pair (hereinafter referred to as Kbp) containing the LT gene was separated. T4 DNA polymerase (PL Inc.) was reacted with the DNA fragment to change the termini of the product to flush ends. Thereafter, an ECOR I linker accompanied with ATG of 16 mer (AACATGAATTCATGTT) was ligated therewith by T4 DNA ligase for adjusting the frame. After T4 DNA ligase was inactivated by heat treatment at 65°C for 10 minutes, digestion was carried out with restriction enzyme EcoRI, and a DNA fragment of 0.6 Kbp containing the LT gene linked with the linker was separated by agarose electrophoresis.

The plasmid ptrp781 described in T. Kurokawa et al., Nucl. Acids Res. 11, 3077 (1983) was cleaved by restriction enzyme EcoRI, and the phosphate of the 5′-terminus was removed by alkaline phosphatase treatment. The thus obtained DNA fragment was mixed with the LT DNA fragment of 0.6 Kbp linked with the EcoRI linker accompanied with ATG, and T4 DNA ligase was allowed to act on the mixture, whereby human LT expression vector pTB622 for Escherichia coli was constructed in which the LT gene was inserted downstream of the tryptophan promoter.

(4) Expression of Human LT (21-171) in Escherichia coli Cells, Extraction from Cells and Purification

Escherichia coli strain described in Reference Example 3 was transformed by using plasmid pTB 622 prepared as described above.

The transformant thus obtained was cultivated in 2.5 l of M9-CA medium [the experimental textbook (1), p. 69] at 37°C for 4 hours. After 25 μg/ml of indolylacrylic acid was added thereto, cultivation was further continued for 4 hours. The cells were collected and suspended in 100 ml of Tris-hydrochloric acid buffer (pH 7.5) containing 0.01% lysozyme and 10% sucrose. The suspension was reacted at 5°C for 1 hour, and then treated with ultrasonication for 5 minutes under ice cooling. After centrifugation, the extract was added to a DEAE-Sepharose CL-6B column (Pharmacia) equilibrated with 5 mM phosphate buffer (pH 8.0), and washed with the same buffer, followed by elution with the same buffer containing 0.1 M NaCl to provide a roughly purified solution having a specific activity of 7.8 X 10⁵ U/mg.

After adjusting to pH 6.0 by hydrochloric acid, the roughly purified solution described above was added to a Blue Sepharose CL-6B column equilibrated with 5 mM phosphate buffer (pH 6.0) containing 0.1 M NaCl, and thoroughly washed, followed by elution with 5 mM phosphate buffer (pH 8.0) containing 0.5 M NaCl. The specific activity of the eluate was 7.4 X 10⁵ U/mg.

The eluate was further subjected to gel filtration by a Sephacryl S-200 column equilibrated with 5 mM phosphate buffer (pH 7.3) to obtain a purified solution having a specific activity of 1.6 X 10⁷ U/mg.

Example 1

Preparation of Mouse Anti-Human LT Monoclonal Antibody

(1) Immunization

To 200 μg of the purified human LT sample described in Reference Example 4-(4) in 1 ml of a physiological saline, the same volume of Freund's complete adjuvant was added and fully emulsified. The emulsion was then administered to BALB/c mice (♀, n = 10 : 20 μg/0.2 ml/mouse) intraperitoneally and subcutaneously at their backs, and supplemental immunization was carried out at an interval of 3 weeks. After the supplemental immunization was conducted 4 times, the same LT antigen solution (50 μg/0.1 ml of physiological saline/mouse) was intravenously administered to the individual which showed the highest serum antibody titer after 2 weeks.

(2) Cell Fusion

The spleen was taken out of the mouse after 3 days from the final immunization, and a spleen cell suspension containing about $10^8$ cells was prepared by a conventional method. Then, 2 X $10^7$ mouse myeloma cells (P3UI) were added thereto, and cell fusion was conducted by using PEG 6000 according to the method of Köler and Milstein [Nature, 256, 495 (1975)].

After completion of the fusion, the cell mixture was suspended in so-called HAT medium containing hypoxanthine, aminopterin and thymidine, and cultivated for 10 days. Then, immediately after selection of parent cells was completed, HAT medium was substituted for HT medium from which aminopterin was eliminated, and the cultivation was continued.

(3) Selection and Cloning of Hybridoma

The antibody titer of hybridoma culture supernatants was determined by the ELISA using a microplate in which purified human LT was adsorbed on a solid phase. After 10 to 20 days from the fusion, hybridomas and an antibody which could bind to human LT were observed. The hybridomas having particularly high binding activity were cloned by the limiting dilution method.

Similarly, the cloned hybridoma culture supernatants were screened by the ELISA, and the supernatants having high binding activity to human LT were selected. For these supernatants, the neutralization ability against the human LT activity was measured by using the test of the cytotoxic activity against L929 cells described in Reference Example 1. Namely, to 100 units/ml of human LT, the same amount of the hybridoma culture supernatant was added and reacted for 1 hour, followed by the test of the cytotoxic activity against L929.

As a result, there were obtained MoAB-producing hybridomas LT3-11 and LT3-135 which were capable of linking with human LT and neutralizing the cytotoxic activity against them. Their immunoglobulin class and subclass were examined by the Ouchterlony test. They were found to belong to IgG$_{2b}$ and IgG$_{2a}$, respectively.

(4) Preparation of Monoclonal Antibody

The cloned hybridoma cells were cultivated in Iskove-Ham mixture medium (I-H medium) containing 10% FCS at 37°C by using a 5% $CO_2$ incubator to obtain an antibody from the supernatant thereof.

On the other hand, in order to obtain a large amount of antibody, 5 X $10^6$ hybridoma cells were intraperitoneally inoculated into a mouse to which 0.5 ml of mineral oil had been intraperitoneally administered in advance. After about 10 to 15 days, retention of ascites was observed. .

The antibody was purified by fractionation with 45-50% saturated ammonium sulfate and DEAE-cellulose and protein A column chromatography, according to a conventional method.

Example 2

Preparation of Rabbit Anti-Human LT-C-Terminal Peptide Antibody

(1) Preparation of Immunogen

A 10 mg/5 ml aqueous solution of the human LT-C-terminal peptide (152-171) prepared by using a peptide synthesizer (Model 430A manufactured by Applied Biosystems) according to a known solid phase synthesis method was added to a 40 mg/5 ml aqueous solution of BTG. After slight ultrasonication, 1 ml of a 2% GLA solution was gently added dropwise thereto under ice cooling and reacted for 5 hours. The mixture was dialyzed 3 times against every 3 l of a physiological saline, and stored under a frozen condition for use as an immunogen.

(2) Immunization

To 4 mg of the peptide-BTG conjugate in 1.5 ml of a physiological saline, the same volume of Freund's complete adjuvant was added. The mixture was subcutaneously administered to rabbits (♂, n = 3: 1.3 mg/1 ml/rabbit) at their backs and hind-limb palms to initiate immunization. Supplemental immunization was carried out by inoculating the immunogen to which the same volume of Freund's incomplete adjuvant was added, 5 times at 4-week intervals.

(3) Preparation of Antibodies

After 7 to 10 days from the final immunization, the blood is collected from the aural veins to obtain the anti-serum by centrifugation.

Specific antibodies were prepared by applying the above-mentioned serum to salting out and column chromatography and further purifying the obtained antibody IgG fraction by affinity chromatography using an insolubilized human LT-cellulofine column, according to a known method. That is to say, the rabbit anti-human LT-IgG fraction was added to a human LT-linked column equilibrated with 0.02 M borate buffer (pH 8.0) containing 0.15 M NaCl, and thoroughly washed, followed by elution with 0.02 M glycine-hydrochloric acid buffer (pH 2.3). Thus, specific neutralizing antibodies LT-R1, LT-R2 and LT-R3 having high affinity for human LT were obtained.

Example 3

ELISA for Human LT

(1) Preparation of Antibody-Bound Solid Phase

Each of the purified antibodies LT3-11 and LT-R2 obtained in Examples 1 and 2 was diluted to a concentration of 10 μg/ml with phosphate buffered saline (hereinafter referred to as PBS) (pH 7.7), and added to a microplate having 96 wells in an amount of 100 μl/well. After standing overnight at 5°C, the antibody solution was removed therefrom and 100 μl of PBS containing 2% BSA and 0.05% thimerosal was added thereto. The resulting solid phases were stored in a cold place until they were used.

(2) Preparation of Biotin-Labeled Antibodies

To 1 ml of each of PBS solutions containing human LT specific antibodies LT3-11 and LT-R2 in an amount of 1 mg/1ml, respectively, 20μl of a 0.5 mg/ml ethanol solution of N-hydroxysuccinimide-biotin (NHS-biotin)(Funakoshi Chemicals) was gently added dropwise and reacted with stirring. After the reaction was conducted at room temperature for 2 hours, the reaction mixture was applied to a Sehpadex G-25 column equilibrated with PBS to remove unreacted NHS-biotin. From the protein fractions thus obtained, the fraction showing the highest antibody activity was collected to obtain the biotin-labeled antibodies.

(3) Determination of Purified Human LT

The purified human LT obtained in Reference Example 4 was diluted with 2% BSA-containing PBS, and 100 μl of the dilute solution was added to each well of the antibody-bound microplate prepared in (1) described above, followed by reaction at room temperature for 2 hours. After completion of the reaction, the plate was washed with PBS containing 0.1% Tween (hereinafter referred to as Tw-PBS). Then, 100 μl of a 1000-fold dilute solution of the biotin-labeled antibody prepared in (2) was added thereto. After the reaction was conducted at room temperature for 2 hours, the plate was washed with Tw-PBS again, and then 100 μl of a 500-fold dilute solution of HRP-labeled avidin (Funakoshi Chemicals) was added thereto, followed by standing at room temperature for 1 hour. After completion of the reaction, the microplate was thoroughly washed with Tw-PBS, and 100 μl of an enzyme substrate solution (0.1 M citrate buffer containing 40 mM o-phenylenediamine and 6 mM hydrogen peroxide, pH 5.5) was added thereto, followed by color-developing reaction for 10 minutes. After the reaction was terminated with 1 N $H_2SO_4$, the absorbance was measured at 492 nm.

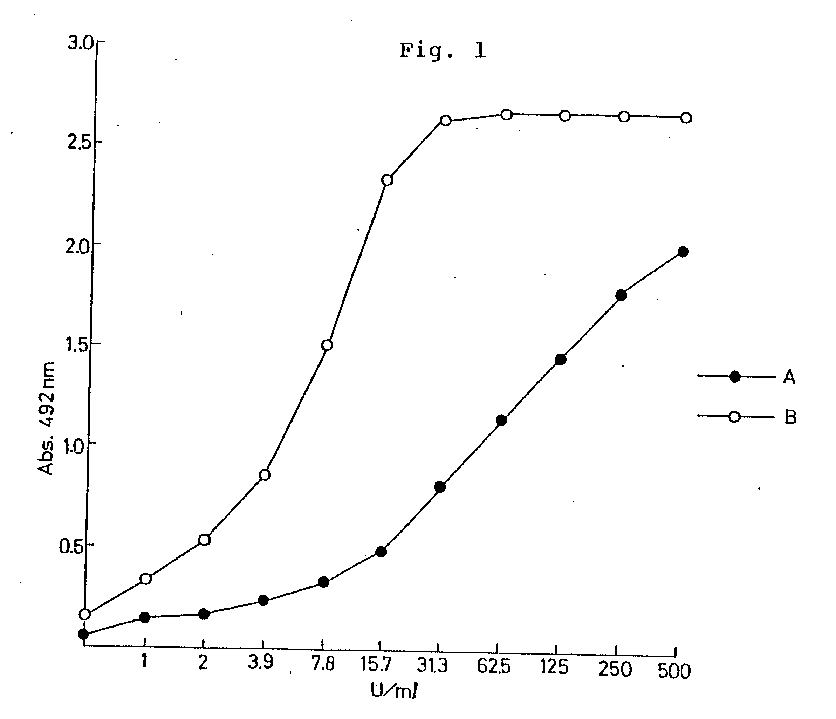

The human LT standard curves shown in Fig. 1 indicate a comparison of the results obtained by the system A using LT3-11 as a solid phase antibody and LT-R2 as a labeled antibody with those obtained by the system B using LT-R2 as a solid phase antibody and LT3-11 as a labeled antibody. According to the system B, about 2 units/ml, 0.1 ng/ml or 10 pg/well of human LT could be determined.

(4) Determination of Human LT in Animal Cell Medium and Cell Extract

For natural human LT in the culture supernatant of activated human peripheral blood lymphocytes obtained in Reference Example 2 and recombinant human LT in the crude extract from Escherichia coli obtained in Reference Example 4-(4), the ELISA according to the system B described in the above (3) was compared with the bioassay for determining the cytotoxic activity against L929 cells described in Reference Example 1.

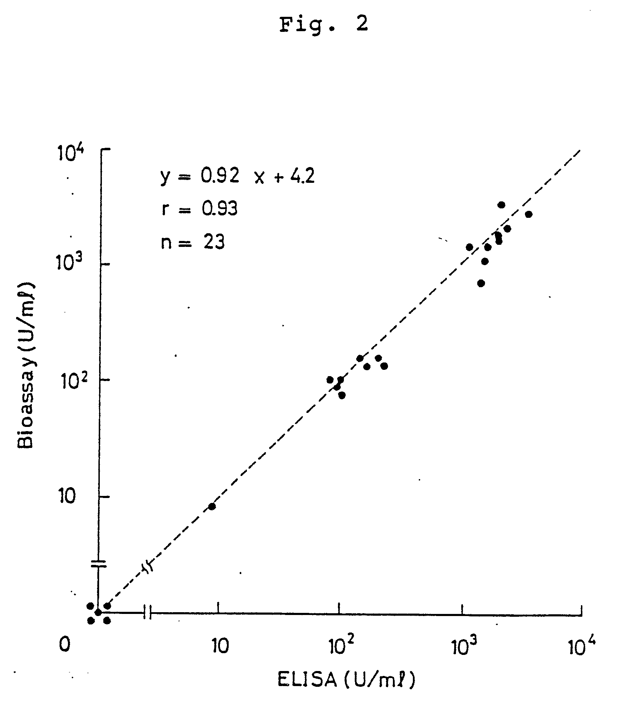

Fig. 2 shows the results of comparison of 23 specimens. The correlation coefficient (r) was 0.93. The determinations by the ELISA according to the present invention were closely related with the biological activity.

## Claims

1. A hybridoma obtainable by fusing a myeloma cell with a spleen cell or a lymphonodus cell of an animal immunized with an N-terminal-deleted human lymphotoxin, having an ability to produce a monoclonal antibody against human lymphotoxin.

2. The hybridoma according to claim 1, wherein the N-terminal-deleted human lymphotoxin is a recombinant human lymphotoxin having a sufficient number of the following amino acid sequence:
H-(Met)n-$R_1$-$R_2$-Ala-His-Ser-Thr-Leu-Lys-Pro-Ala-Ala-His-Leu-Ile-Gly-Asp-Pro-Ser-Lys-Gln-Asn-Ser-Leu-Leu-Trp-Arg-Ala-Asn-Thr-Asp-Arg-Ala-Phe-Leu-Gln-Asp-Gly-Phe-Ser-Leu-Ser-Asn-Asn-Ser-Leu-Leu-Val-Pro-Thr-Ser-Gly-Ile-Tyr-Phe-Val-Tyr-Ser-Gln-Val-Val-Phe-Ser-Gly-Lys-Ala-Tyr-Ser-Pro-Lys-Ala-Thr-Ser-Ser-Pro-Leu-Tyr-Leu-Ala-His-Glu-Val-Gln-Leu-Phe-Ser-Ser-Gln-Tyr-Pro-Phe-His-Val-Pro-Leu-Leu-Ser-Ser-Gln-Lys-Met-Val-Tyr-Pro-Gly-Leu-Gln-Glu-Pro-Trp-Leu-His-Ser-Met-Tyr-His-Gly-Ala-Ala-Phe-Gln-Leu-Thr-Gln-Gly-Asp-Gln-Leu-Ser-Thr-His-Thr-Asp-Gly-Ile-Pro-His-Leu-Val-Leu-Ser-Pro-Ser-Thr-Val-Phe-Phe-Gly-Ala-Phe-Ala-Leu-OH
wherein $R_1$ is Pro or Phe, $R_2$ is a peptide chain represented by Ala-Gln-Thr-Ala-Arg-Gln-His-Pro-Lys-Met-His-Leu or a part thereof, and n is 0 or 1, to produce a monoclonal antibody against human lymphotoxin.

3. The hybridoma according to claim 2, wherein the recombinant human lymphotoxin has the amino acid sequence described.

4. The hybridoma according to claim 1, which is mouse-mouse hybridoma LT3-11 or LT3-135.

5. An anti-human lymphotoxin monoclonal antibody produced by the hybridoma according to claim 1.

6. A mouse IgG monoclonal antibody which is produced by mouse-mouse hybridoma LT3-11 or LT3-135 according to claim 4, and has an ability to neutralize cytotoxicity of human lymphotoxin.

7. The hybridoma according to claim 1, which produces an anti-human lymphotoxin monoclonal antibody which has an ability to neutralize cytotoxicity of human lymphotoxin.

8. An anti-human lymphotoxin antibody obtainable from an animal immunized with a human lymphotoxin-C-terminal peptide.

9. The anti-human lymphotoxin antibody according to claim 8, wherein the human lymphotoxin-C-terminal peptide is a synthetic peptide having the following amino acid sequence:
<H-R-Ser-Thr-Val-Phe-Phe-Gly-Ala-Phe-Ala-Leu-OH
wherein R is a peptide chain represented by Leu-Thr-Gln-Gly-Asp-Gln-Leu-Ser-Thr-His-Thr-Asp-Gly-Ile-Pro-His-Leu-Val-Leu-Ser-Pro or a part thereof.

10. The anti-human lymphotoxin antibody according to claim 9, which is rabbit antibody LT-R1, LT-R2 or LT-R3 having an ability to neutralize cytotoxicity of human lymphotoxin.

11. The anti-human lymphotoxin antibody according to claim 8 having an ability to neutralize cytotoxicity of human lymphotoxin.

12. An immunochemical method for assaying human lymphotoxin in a test solution which comprises reacting the test solution with an anti-human lymphotoxin antibody held on a carrier, further reacting an anti-human lymphotoxin labeled with a labeling agent therewith, and then determining an activity of the labeling agent held or not held on the carrier, wherein the antibody held on the carrier and the antibody labeled with the labeling agent are two kinds of antibodies wherein their antigen-determining sites do not overlap each other, one of which is an antibody reactive to a human lymphotoxin-C-terminal peptide and the other is an anti-human lymphotoxin monoclonal antibody.

13. A method for producing a hybridoma having an ability to produce a monoclonal antibody against human lymphotoxin, which comprises fusing a myeloma cell with a spleen cell or a lymphonodus cell of an animal immunized with an N-terminal -deleted human lymphotoxin.

Fig. 1

EP 0 335 263 A2

Fig. 2